# EUROPEAN PATENT APPLICATION

(11) **EP 4 186 374 A1**
(43) Date of publication of application: **31.05.2023**
(21) Application number: 21210184.4
(22) Date of filing: 24.11.2021
(51) Int. Cl.: A23K 10/30, A01N 65/08, A23K 20/111, A23L 33/105, A61K 8/9789, A61K 36/185, C12C 3/00, C12C 3/08, C12C 5/02

(54) **BITTER ACIDS CONCENTRATE, METHOD OF OBTAINING THE SAME AND USE THEREOF**

(71) Applicant: Herbolea Biotech S.p.A., 50019 Sesto Fiorentino (IT)
(72) Inventor: VENTURINI DEL GRECO, Giovanni, 50121 Firenze (IT)
(74) Representative: Zaccaro, Elisabetta

(57) **Abstract**

The invention relates to a bitter acids concentrate, method of obtaining the same and use thereof.

## Description

### FIELD OF THE INVENTION

The invention relates to a bitter acid concentrate, method of obtaining the same and use thereof.

### BACKGROUND

The cultivation of the hop plant *(Humulus lupulus L.)* is traditionally associated with beer brewing. During the Middle Ages it was used for the first time in the brewing process as a natural flavouring and antibacterial agent.

Adding hops to beer reduces the growth of Lactobacillus, the main beer contaminant, thus preserving the yeast performance, undesirable losses in ethanol, and the formation of undesirable off-flavours. The preservative properties of hops have been investigated for many years and, despite some reports on the anti-bacterial activity of hop oil, the bitter acids seem to be the main active compounds. The target bacteria are Gram-positive species, such as Lactobacillus, Streptococcus, Staphylococcus, Micrococcus, and Bacillus. In contrast, Gram-negative bacteria, such as *Escherichia coli,* are either resistant or only affected at very high concentrations of hop acids.

Hops have also been used in folkloric medicine to treat sleeping disorders, to activate gastric function, to alleviate nervousness and to be applied in cases of earache or toothache. In fact, hops have anti-inflammatory, antiseptic, antidiuretic, hypnotic, and sedative properties.

Furthermore, hop bitter acids, like several natural compounds, have been identified as an interesting class of compounds for the use in cancer chemotherapy, cancer chemoprevention as well as for the use as antimicrobial substances.

Nowadays about 95% of world-wide cultivated hops is destined for brewing purposes, while the remaining 5% is used in the field of phytopharmaceuticals and dietary supplements to treat sleep disorders as well as menopausal symptoms.

Hops contain a class of compounds of interest, namely bitter acids, consisting of two groups: the alpha-acids (or humulones) and the beta-acids (or lupulones), which are both characterized as prenylated phloroglucinol derivatives. The relative amounts of alpha-acids and beta-acids, as well as the concentrations of individual representatives, depend strongly on the hop strain and the growing conditions.

In general, lupulone has greater antimicrobial activity than humulone, which is, in turn, more active than iso-humulone. It appears that the more prenyl groups (three in the beta-acids) are present, the stronger the bacteriostatic action is. However, the role of iso-alpha-acids in beer preservation is of great value, since they represent quantitatively the main contribution of hops to beer. In all studies, hop acids may behave as either bacteriostatic substances or bactericides, depending on the conditions employed.

The alpha-acids are the most important constituents of hops and represent up to 20% of the dry weight of the hop cones. They play some important roles in brewing like improving foam stability, suppressing gushing, and contributing to the preservation of beer. However, their main role in beer production is the isomerization during the boiling of wort at a pH of 4.0-5.5, thereby forming the iso-alpha-acids (or isohumulones), the largest contributor to beer bitterness. In practice, due to poor extractability from hops, poor solubility of the alpha-acids in the broth and incomplete isomerization, a final alpha-acid utilization of only 25-35% is achieved during the brewing process.

Iso-alpha-acids represent more than 80% of all hop-derived components in beer. The high concentrations of these compounds in the final beverage and their relatively low bitter taste thresholds support their function as major bitter agents in beer. Furthermore, they possess tensioactive properties, thereby stabilizing the beer foam, and preserving beer against micro-organisms.

Beta-acids, on the other hand, are relatively insoluble into water and therefore do not contribute much to the perceived bitterness of beer. They are extremely sensitive to oxidation, giving rise to several oxidized compounds and derivatives. One particular and most important oxidative reaction leads to formation of the highly stable hulupones. In contrast to the non-bitter beta-acids, hulupones have a very bitter taste (threshold concentration of 7.9 µmol/L) and can be present in beer in quantities of few mg/L.

For the brewing industry, deterioration of hops cones as a function of time has always been a main issue. This is in fact accompanied by the development of a strong odour that is generally not welcomed by brewers. To prevent this, hops are rapidly dried after harvesting, pelleted, and stored in airtight bags, preferably at low temperatures.

Today, next to the use of pellets, many beers can be rendered bitter-tasting with hop extracts, which lead to the benefits of an increased utilization of brewing principles, increased stability, improved uniformity, more precise dosing and avoiding the aroma of pellets which is not appreciated by brewers. Additionally, a smaller volume of material per gram of bitter acids is required in comparison to the hop plant, thus reducing costs related to hop storage, reducing losses due to the absorption of the wort by the hops' cellulosic material, and waste disposal.

Hop acids can be separated from the vegetative material by extraction with solvents of different polarity. Hops are typically extracted by means of supercritical or liquid carbon dioxide (US4218491A and EP0679419B1), yielding a dark-green or a yellow-golden paste, depending on the extraction conditions and the hop variety. This paste contains high levels of alpha-acids, beta-acids and waxes without the more polar tannins, hard resins, and salts.

Supercritical CO₂ requires hops to be dried. By drying hops, part of the most volatile compounds, such as some terpenes which are responsible for fragrance (i.e. Myrcene and Pinene), are inevitably lost. Furthermore, additional volatile terpenes are lost during supercritical CO₂ extraction. Additionally, supercritical CO₂ extraction is a capital- and labour-intensive process. Due to high pressure that can lead to explosion, the process must be performed with expensive equipment and managed by highly skilled personnel. Furthermore, supercritical CO₂ extraction requires further post-treatments to separate alpha acids from beta acids.

US4212895 provides a method to obtain a high-content iso-alpha-acid concentrate from a first extract containing alpha acids and beta acids. The latter is obtained from dried hops using liquid CO₂ at a sub-critical temperature of not less than -5°C. The further isomerization is performed by boiling an alkaline solution of the extract. This boiling step is utilized to remove the hop oil (terpenes). The beta-acids are removed by acidifying the solution of iso-alpha-acid and filtering off the precipitated beta-acids. The iso-alpha-acid preparation can be added to beer after fermentation to bright beer after final filtration to provide bitterness.

US4590296A provides a method of separating beta-acids from alpha-acids starting from a CO₂ extract containing beta-acids and alpha-acids without using organic solvents, said method consisting of the hop extract having a pH of about 9.6 to about 13 by bubbling CO₂ through the extract to lower the pH to about 8.5 to about 9.5 to precipitate the beta-acids and then isolating the pure solid beta-acids from the pure alpha-acids which remain in the aqueous extract.

EP0020087A1 describes a method of purifying aqueous solutions of iso-alpha-acid salts, obtained by treating a commercial alpha-acid hexane extract with a strong base, by removing beta-acids therefrom. The solution is brought to an iso-alpha-acid concentration of 0.5% to 10%w/_{w}, particularly 0.5% to 5%w/_{w}, then reducing the pH to a value within the range 7 to 10, preferably 8 to 9 to form a filtered precipitate of beta-acids. Preferably pH reduction is obtained by bubbling carbon dioxide through the solution. The separated iso-alpha-acid solution may be concentrated to an extent that a phase separation takes place into two aqueous phases one of which has a higher iso-a-acid concentration than the other.

WO1995020037A1 relates to the production of concentrated hop materials, and more particularly to the production of a purified, iso-alpha-acid concentrate by using a metal salt isomerizing agent capable of converting alpha-acids in a CO₂ hop extract to iso-alpha-acid metal salts. A chemical mixture comprising a first organic phase and a first aqueous phase is obtained. Said first organic phase comprises iso-alpha-acid metal salts, beta-acids, and hop oils. The iso-alpha-acids are then isolated from the rest through different purification steps.

### SUMMARY OF INVENTION

The Applicant noted that methods to obtain bitter acids concentrates are known, but they consist in long and expensive operations. Furthermore, the Applicant also noted that it does not seem to exist a "green" method to directly process fresh hops, with no need of drying, and purify alpha-acids from beta-acids, without making use of organic solvents.

Therefore, the Applicant thought that a method to obtain a bitter acids concentrate, advantageously enriched in alpha acids, would be desirable and that a "green" technique making no use of organic solvents and expensive equipment to purify alpha-acids could represent a valuable and environmentally friendly alternative.

An object of the present invention is to provide a method to obtain a bitter acids concentrate, advantageously enriched in alpha acids, making no use of organic solvents.

Therefore, the present invention relates, in a first aspect, to a method for preparing a bitter acids concentrate comprising more than 50% by dry weight, with respect to the total dry weight of the concentrate, of bitter acids, comprising the steps of:
a) providing a lipid extract from a starting biological material containing bitter acids;
b) mixing said lipid extract with an alkaline aqueous solution to form a mixture having a pH of at least 9;
c) separating from the mixture of step b) an aqueous phase containing bitter acid salts; and
d) obtaining said bitter acids concentrate.

Surprisingly, the Applicant has found out that by applying the described method is possible to obtain bitter acid concentrates having a high content of bitter acids and one or more advantageous compositional features, making no use of organic solvents and expensive equipment/reagents, that represents an easily adjustable, simple, valuable, and environmentally friendly alternative to the methods of the prior art.

The applicant surprisingly also noted that by applying the described method is also possible to obtain, in an efficient manner, bitter acids concentrates that advantageously have a surprisingly high content of alpha acids and a relatively low content of beta acids, with no appreciable alkaline hydrolysis of the triglycerides of the vegetable oil contained in the lipid extract.

In a further aspect, the present invention relates also to a bitter acids concentrate obtainable by means of the method according to said first aspect and to a bitter acids concentrate comprising more than 50% by dry weight, with respect to the total dry weight of the concentrate, of bitter acids and one or more of the following features:
- a weight ratio (alpha acids) : (beta acids) of at least 3:1, preferably of more than 10:1, even more preferably of more than 20:1;
- the bitter acids concentrate comprises at least, preferably more than, 1.5% by dry weight, with respect to the total dry weight of said concentrate, of xanthohumol;
- the bitter acids concentrate comprises less than 10 % by weight, preferably less than 5% by weight, more preferably less than 3% by weight, on total concentrate dry weight, of total beta acids;
- the bitter acids concentrate comprises at least 30% by weight, more preferably at least 50% by weight, even more preferably at least 60% by weight, even more preferably at least 65 % by weight, with respect to the total dry weight of the concentrate, of alpha-acids;
- a content of essential oil, such as humulene, myrcene, and caryophyllene, of less than 1% by weight;
- the bitter acids concentrate has a beta acids content lower than that of the starting biological material;
- the bitter acids concentrate comprises not more than 0.0001% per cent by weight of one or more organic solvents selected from a group consisting of acetone, benzene, butane, chloroform, cyclohexane, dichloromethane, ethanol, ethyl acetate, ethylbenzene, heptane, hexane, isobutane, isopropanol, methanol, pentane, propane, toluene, m-xylene, o-xylene, and p-xyleneheptane; and
- the bitter acids concentrate comprises less than 5% of iso-alpha acids.

The advantages of these bitter acids concentrates according to the present invention have been disclosed in relation to the method according to the first aspect of the present invention and are not herewith repeated.

Thanks to its compositional properties, said bitter acids concentrates might be advantageously used in a brewing process and/or for preparing a pharmaceutical product, a nutraceutical product, a cosmetic product, a food product, a feed product, an antimicrobial, an antibacterial, an insecticide, or a biopesticide containing bitter acids.

### BRIEF DESCRIPTION OF THE DRAWINGS

Figure 1 shows the HPLC trace of the lipid extract of Example 1;
Figure 2 shows the HPLC trace of the lighter oily phase obtained from the separation step of Example 1;
Figure 3 shows the HPLC trace of the precipitate obtained from the heavier aqueous phase obtained from the separation step of Example 1;
Figure 4 shows the HPLC trace of the lipid extract of Example 2;
Figure 5 shows the HPLC trace of the lighter oily phase obtained from the separation step of Example 2;
Figure 6 shows the HPLC trace of the precipitate obtained from the heavier aqueous phase obtained from the separation step of Example 2.

### DETAILED DESCRIPTION OF INVENTION

The present invention relates, in a first aspect, to a method for preparing a bitter acids concentrate comprising more than 50% by dry weight, with respect to the total dry weight of the concentrate, of bitter acids, comprising the steps of:
a) providing a lipid extract from a starting biological material containing bitter acids;
b) mixing said lipid extract with an alkaline aqueous solution to form a mixture having a pH of at least 9;
c) separating from the mixture of step b) an aqueous phase containing bitter acid salts; and
d) obtaining said bitter acids concentrate.

Surprisingly, the Applicant has found out that by applying the described method is possible to obtain bitter acid concentrates having a high content of bitter acids and one or more advantageous compositional features, making no use of organic solvents and expensive equipment/reagents that represent an easily adjustable, simple, valuable, and environmentally friendly alternative to the methods of the prior art.

The applicant surprisingly also noted that by applying the described method is also possible to obtain bitter acids concentrates in an efficient manner that advantageously have a surprisingly high content of alpha acids and a relatively low content of beta acids, with no appreciable alkaline hydrolysis of the triglycerides contained in the lipid extract.

Within the framework of the present description and in the subsequent claims, except where otherwise indicated, all the numerical entities expressing amounts, parameters, percentages, and so forth, are to be understood as being preceded in all instances by the term "about". Also, all ranges of numerical entities include all the possible combinations of the maximum and minimum values and include all the possible intermediate ranges, in addition to those specifically indicated herein below.

Listed below are definitions of various terms used to describe this invention. These definitions apply to the terms as they are used throughout this specification and claims, unless otherwise limited in specific instances, either individually or as part of a larger group.

Unless defined otherwise, all technical and scientific terms used herein generally have the same meaning as commonly understood by one of ordinary skill in the art to which this invention belongs. Generally, the nomenclature used herein and the laboratory procedures in cell culture, molecular genetics, organic chemistry, and peptide chemistry are those well-known and commonly employed in the art.

As used herein, the articles "a" and "an" refer to one or to more than one (i.e. to at least one) of the grammatical object of the article. By way of example, "an element" means one element or more than one element.

As used herein, the terms "lipid" or "lipids" include, but are not limited to, olive oil, coconut oil, vegetable oil, milk, butter, liposomes, glycerine, polyethylene glycol, ethyl acetate, d-limonene, liquid paraffin, butylene glycol, propylene glycol, ethylhexyl palmitate.

As used herein, the term "about" will be understood by persons of ordinary skill in the art and will vary to some extent on the context in which it is used. As used herein when referring to a measurable value such as an amount, a temporal duration, and the like, the term "about" is meant to encompass variations of ±20% or ±10%, including ±5%, ±1 %, and ±0.1% from the specified value, as such variations are appropriate to perform the disclosed methods.

As used herein, with the expression "bitter acids" is meant a class of prenylated polyketides synthesized in lupulin glands of the hop plant (Humulus lupulus), which advantageously are important contributors to the bitter flavour and stability of beer. They include alpha acids, iso-alpha acids, and beta acids;
as used herein, the expressions "alpha acid" and "alpha acids" include compounds of general formula (I) 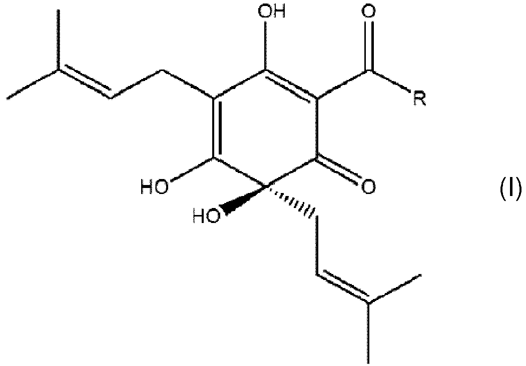 wherein R is an alkyl group, for example CH(CH₃)₂, CH₂CH(CH₃)₂, or CH(CH₃)CH₂CH₃. Specific examples of alpha acids are humulone, adhumulone, cohumulone, posthumulone, and prehumulone.

As used herein, with the expressions "iso-alpha acid" and "iso-alpha-acids" is meant a class of compounds derived from the isomerization of alpha acids and including compounds of general formula (II) and (III) 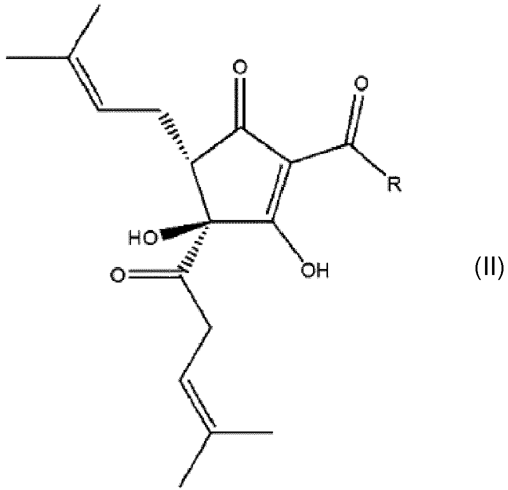 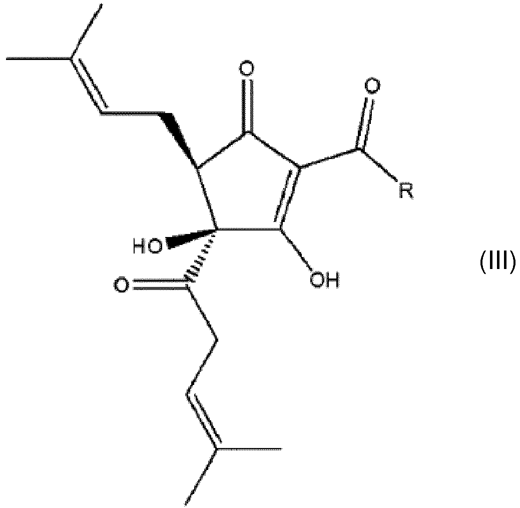 wherein R is an alkyl group, for example CH(CH₃)₂, CH₂CH(CH₃)₂, or CH(CH₃)CH₂CH₃. Specific examples of iso-alpha acids are cis- and trans-isohumulone, cis- and transisocohumulone, cis-and trans-isoadhumulone.

As used herein, the expressions "beta acid" and "beta-acids" include compounds of general formula (IV) 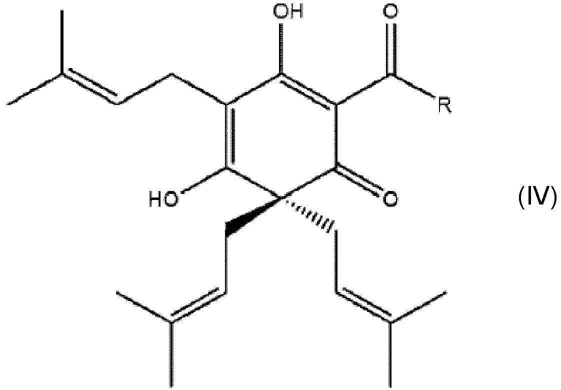 wherein R is an alkyl group, for example CH(CH₃)₂, CH₂CH(CH₃)₂, or CH(CH₃)CH₂CH₃. Specific examples of beta acids are co-lupulone, ad-lupulone, lupulone, prelupulone, and postlupulone.

The present invention may present in one or more of the above aspects one or more of the characteristics disclosed hereinafter.

Further features and advantages of the invention will appear more clearly from the following description of some preferred embodiments thereof, made hereinafter by way of a nonlimiting example with reference to the following exemplary examples.

The method according to the present invention comprises the step a) of providing a lipid extract from a starting biological material containing bitter acids.

Preferably, said lipid extract comprises no more than 1.5% by dry weight of beta-acids. Preferably, said lipid extract comprises no more than 1.5% by dry weight of co-lupulone, ad-lupulone, lupulone, prelupulone, and postlupulone.

Preferably, said lipid extract comprises at least 1% by dry weight, of alpha-acids.

Preferably, said lipid extract comprises at least 1% by dry weight, of at least one alpha acid selected from the group consisting of: humulone, adhumulone, cohumulone, posthumulone, and prehumulone.

Preferably, said lipid extract has a total alpha-acids content of at least 2% by dry weight, more preferably of at least 3% by dry weight, even more preferably of at least 5% by dry weight.

In a preferred embodiment, the lipid extract of step a) comprises at least 3 % dry weight, with respect to the lipid extract dry weight, of bitter acids and in said lipid extract the weight ratio (alfa acids):(beta acids) is of more than 1:1. In such a case, in the resulting bitter acids concentrate the weight ratio (alpha acids) : (beta acids) is advantageously of at least 3:1, preferably of more than 10:1, even more preferably of more than 20:1.

In a further preferred embodiment of the method of the invention, the lipid extract of step a) comprises at least 3 % by dry weight, with respect to the lipid extract dry weight, of bitter acids and at least 0.2% by of xanthohumol. In such a case, when the mixture of step b) has a pH of at least 11 the resulting bitter acids concentrate advantageously comprises at least 1.5% by dry weight, with respect to the total dry weight of said concentrate, of xanthohumol.

Preferably, the lipid of said lipid extract of step a) is selected from the group consisting of: vegetable oil, milk, butter, liposomes, ethyl acetate, glycerine, d-limonene, caryophyllene, liquid paraffin, mineral oil, paraffin wax, microcrystalline wax, mineral wax, ozokerite, polyethylene, polyoxyethylene and hydrocarbon waxes derived from carbon monoxide and hydrogen, cerosin; cetyl esters; hydrogenated joioba oil, butylene glycol, propylene glycol, polyethylene glycol, liposomes, lecithin, ethylhexyl palmitate, or mixtures thereof.

In another embodiment, the lipid is a vegetable oil.

Preferably, said vegetable oil is selected from the group consisting of: olive oil, coconut oil, sunflower oil, sesame oil, hemp seed oil, Medium-chain triglycerides (MCTs) oil, or a mixture thereof.

In an embodiment, the lipid is olive oil. In another embodiment, the lipid is coconut oil. In yet another embodiment, the lipid is sunflower oil. In another embodiment, the lipid is Medium Tryglicerids Chains (MCTs) oil. In an embodiment, the lipid is milk. In a further embodiment, the lipid is butter. In yet another embodiment, the lipid is a liquid paraffin.

Preferably, the lipid is a vegetable oil having a free-fatty acids (FFA) content below 1%, preferably below 0.5%, even more preferably below 0.1%.

The lipid extract of step a) is obtained from a starting biological material containing bitter acids, preferably said starting biological material is a plant, advantageously selected from the *Humulus* genus of plants, including a pure, a hybrid or a genetically modified variant of said plants. Preferably, said plant selected from the *Humulus* genus of plants, belongs to the species *H. lupulus.*

Preferably said starting biological material is any part of said plant, including its flowers, leaves, stems; more preferably said starting biological material is a flower of a plant containing bitter acids, advantageously selected from the *Humulus* genus of plants, preferably belonging to the species *H. lupulus.*

In a preferred embodiment, the lipid extract of step a) is obtained by putting in contact a starting biological material with a vegetable oil.

Preferably, the lipid extract of step a) is obtained from a starting biological material by means of the steps of:
I. comminuting a starting biological material;
II. mixing the comminuted material with lipids to obtain a mixture;
III. agitating the mixture at a temperature range of 1 to 90 °C; and
IV obtaining a lipid extract from the mixture of step III.

Preferably, said starting biological material is a plant, advantageously selected from the *Humulus* genus of plants, including a pure, a hybrid or a genetically modified variant of said plants. Preferably, said plant selected from the *Humulus* genus of plants, belongs to the species *H. lupulus.*

Preferably, said starting biological materials is any part of plant, including its flowers, leaves, stems; more preferably said starting biological material is a flower of a plant containing bitter acids, advantageously selected from the *Humulus* genus of plants, preferably belonging to the species *H. lupulus.*

In said step I., the starting biological material is milled to increase the surface contact. Then water and lipids are added to the starting biological material to form a homogeneous mixture or slurry; the mixture may be agitated through stirring or other agitation methods preferably for at least 30 min. Ultrasound/sonication or microwaves or steam explosion may advantageously be used before or after adding lipids to the mixture to reduce the time necessary to achieve biological material dissolution and alpha-acids lipid-extraction yield.

The mixture obtained is then separated for example via density separation (i.e. centrifugation) or pressing (French press) and/or filtration to recover a lipid fraction highly enriched with bitter acids.

Preferably, said starting biological material is in form of pellet and has a moisture content of about 5-10%.

Preferably, said starting biological material can be used in said step I. of the method according to the invention either fresh or dried. In an embodiment, the starting biological material is newly harvested and contain high level of moisture; in such a case addition of extra water to the starting biological material is unnecessary.

Preferably, the starting biological material has a total bitter acids content of at least 5% by weight, more preferably of at least 10 % by weight, even more preferably of at least 15% by weight, even more preferably of at least 20% by weight.

Preferably, said starting biological material contains at least 10% alpha-acids in weight.

Preferably, in step II. the lipids have been neutralized prior being added to the mixture.

Preferably the lipids are added before or during the step of comminuting the biological material.

Preferably, enzymes are added to the mixture of said step II.

Preferably, said enzymes are one or more independently selected from the group consisting of oxidoreductases, transferases, hydrolases, lyases, isomerases, and ligases, cellulase, hemicellulase, xylanase, glucanase, beta-glucanase, pectinase, glucuronyltransferase, lipase, amylase, alpha-amylase, beta-amylase, phospholipase, arabanase, galacto-, betamannanase, protease, phytase, humulone synthase.

In an embodiment, said enzyme is cellulase. In another embodiment, said enzyme is betaglucosidase. In another embodiment, said enzyme is hemicellulase. In another embodiment, said enzyme is xylanase. In yet another embodiment, said enzyme is glucanase. In yet another embodiment, said enzyme is pectinase. In still another embodiment, said enzyme is amylase. In yet another embodiment, said enzyme is lipase or phospholipase. In said another embodiment, said enzyme is glucuronosyltransferase or alcohol dehydrogenase. In yet another embodiment, said enzyme is arabinanase. In still another embodiment, said enzyme is phytase. In a further embodiment, said enzyme is protease.

Preferably, in step II. the temperature varies in the range from 40 to 70 °C. Preferably, in step II. the pH varies in the range from 4 to 6.

Preferably, the mixture of said step III. is degummed with phosphoric acid.

Preferably, said step IV comprises separating the mixture into a lipid phase, an aqueous phase, and a solid phase; wherein the lipid phase comprises the lipid extract.

In an embodiment, in step IV. the mixture is separated by density. In a further embodiment, in step IV. the mixture is separated by pressing and/or filtering.

In a further embodiment, in step IV, the mixture is separated into a lipid phase and a wet solid phase.

In an alternative embodiment, steps I. and II. can be inverted in their order, so that the lipid extract of step a) is obtained from a starting biological material by means of the steps of:
I-i. mixing a starting biological material with lipids to obtain a mixture;
II-i. comminuting the mixture of step I.;
III-i. agitating the mixture at a temperature range of 1 to 90 °C; and
IV-i obtaining a lipid extract from the mixture of step III.

In said alternative embodiment, any one of steps I-i, II-i, III-i, IV-i may have one or more of the preferred characteristics respectively of step II, I, III, and IV above, which can be combined with each other according to the application requirements.

In an embodiment, the lipid extract is recirculated any number of times to achieve higher bitter acids content.

In an embodiment, the lipid extract has a total bitter acids content of at least 1% by weight. In a further embodiment, the lipid extract has a bitter acids content of at least 3% by weight. In yet another embodiment, the lipid extract has a bitter acids content of at least 5% by weight.

In a preferred embodiment of the method according to the present invention, the aqueous phase resulting from said step of separating the mixture into a lipid phase, an aqueous phase, and a solid phase, wherein the lipid phase comprises the lipid extract, can also be used in the production of nutraceutical, antimicrobial, antibacterial products or biopesticides.

The method according to the present invention comprises a step b) of mixing said lipid extract with an alkaline aqueous solution to form a mixture having a pH of at least 9.

Preferably, the alkaline aqueous solution of step b) comprises at least one hydroxide of at least one metal selected from the group consisting of: alkali metal, and alkaline earth metal.

Preferably, the alkaline aqueous solution of step b) comprises NaOH, KOH or a mixture thereof.

Preferably, the alkaline aqueous solution of step b) is 0.1 M NaOH or KOH.

More preferably, the alkaline aqueous solution of step b) is 0.05 M NaOH or KOH.

Preferably, in step b) the alkaline aqueous solution is added to the lipid extract in a weight ratio (alkaline aqueous solution):(lipid extract) of at least 1:1, more preferably of at least 2:1, even more preferably of at least 2.5:1.

Preferably, the mixture of step b) is mixed at 25 °C for a time in the range from at least 5 seconds to less than 60 minutes, more preferably from at least 1 minute to less than 30 minutes, even more preferably from 2 to 10 minutes.

Preferably, the mixture of step b) has a pH value of at most 13.5.

Preferably, in step b) the alkaline aqueous solution has a molarity of NaOH calculated on the base of the total acidity of the lipid extract expressed as moles of KOH required for acidic titration of the lipid extract, so that the ratio NaOH mol / KOH mol is in the range of 5-12.

The method according to the invention comprises a step c) of separating from the mixture of step b) an aqueous phase containing bitter acid salts.

Preferably, in the method according to the invention before step c) at least one salt and/or at least one sugar is added to the mixture of step b).

In an embodiment, said step c) of separating comprises separating from the mixture of step b):
- a lighter oily phase,
- a heavier aqueous phase, wherein the heavier aqueous phase is the aqueous phase containing bitter acid salts.

In this way, two phases are advantageously obtained. The lighter oily phase contains most of beta-acids but is depleted of most of the alpha-acids, whereas the heavier aqueous phase contains bitter acid salts, advantageously an increased amount of alpha-acid salts and a reduced amount of beta-acid salts.

Preferably the heavier aqueous phase is spray dried.

Preferably, in said embodiment the lighter oily phase is distilled to recover low boiling terpenes such as myrcene and humulene. Advantageously, said low boiling terpenes are suitable as components of essential oils, such as for example hop oil, and can be added to the bitter acids concentrate in precise quantity.

The method according to the invention comprises a step d) of obtaining said bitter acids concentrate.

In an embodiment, said step d) comprises:
i. acidifying the aqueous phase of said step c) to a pH of less than 4, thus forming a precipitate comprising bitter acids;
ii. separating the precipitate of step i. from the remaining aqueous phase, wherein said precipitate is said bitter acids concentrate.

In a further embodiment, said step d) comprises:
i. modifying the pH of the aqueous phase of said step c) to a value ranging from 12.5 to 9, thus forming a first precipitate;
ii. separating the first precipitate of step i. from the remaining aqueous phase, wherein said first precipitate contains impurities;
iii. modifying the pH of the remaining aqueous phase of step ii. to a value of less than 11.5, thus forming a second precipitate;
iv. separating the second precipitate from the further remaining aqueous phase, wherein said second precipitate is said bitter acids concentrate.

In a still further embodiment, said step d) comprises drying the aqueous phase, thus forming a dried product, wherein said dried product is said bitter acids concentrate.

Preferably, the bitter acids concentrate obtained from step d) comprises less than 10 % by weight, more preferably less than 5 % by weight, even more preferably less than 3 % by weight, on total concentrate dry weight, of total beta acids.

Preferably, the bitter acids concentrate obtained from step d) has a beta acids content lower than that of the lipid extract of step a).

Preferably, the bitter acids concentrate obtained from step d) has a beta acids content lower than that of the starting biological material.

Preferably, the bitter acids concentrate obtained from step d) comprises at least 30% by weight, more preferably at least 50% by weight, even more preferably at least 60% by weight, with respect to the total dry weight of the concentrate, of alpha-acids.

In a preferred embodiment, the bitter acids concentrate obtained from step d) comprises more than 60% by weight, with respect to the total dry weight of the concentrate, of alpha-acids and not more than 3% by weight, with respect to the total dry weight of the concentrate, of beta-acids.

In a further preferred embodiment, the bitter acids concentrate obtained from step d) comprises more than 60% by weight, with respect to the total dry weight of the concentrate, of alpha-acids and not more than 0.0001% per cent by weight of one or more organic solvents selected from a group consisting of acetone, benzene, butane, chloroform, cyclohexane, dichloromethane, ethanol, ethyl acetate, ethylbenzene, heptane, hexane, isobutane, isopropanol, methanol, pentane, propane, toluene, m-xylene, o-xylene, and p-xyleneheptane.

The Applicant has noted that the bitter acids concentrate obtained with a cheap and solventfree method, is particularly surprising compared to the prior art concentrates, in which a high bitter acid, especially alpha-acids, content is usually achieved by means of supercritical CO₂, a very expensive technique or by treatments that involve the use of organic solvents, the elimination of which may result troublesome and expensive as well.

According to the present invention, a bitter acids concentrate is therefore provided.

In a further aspect, the present invention relates therefore also to a bitter acids concentrate obtainable by means of the method according to said first aspect and to a bitter acids concentrate comprising more than 50% by dry weight, with respect to the total dry weight of the concentrate, of bitter acids and one or more of the following features:
- a weight ratio (alpha acids) : (beta acids) of at least 3:1, preferably of more than 10:1, even more preferably of more than 20:1;the bitter acids concentrate comprises at least, preferably more than, 1.5% by dry weight, with respect to the total dry weight of said concentrate, of xanthohumol;
- the bitter acids concentrate comprises less than 10 % by weight, more preferably less than 5 % by weight, even more preferably less than 3 % by weight, on total concentrate dry weight, of total beta acids;
- the bitter acids concentrate comprises at least 30% by weight, more preferably at least 50% by weight, even more preferably at least 60% by weight, even more preferably at least 65 % by weight, with respect to the total dry weight of the concentrate, of alpha-acids;
- a content of essential oil, such as humulene, myrcene, and caryophyllene, of less than 1% by weight;
- the bitter acids concentrate has a beta acids content lower than that of the starting biological material;
- the bitter acids concentrate comprises not more than 0.0001% per cent by weight of one or more organic solvents selected from a group consisting of acetone, benzene, butane, chloroform, cyclohexane, dichloromethane, ethanol, ethyl acetate, ethylbenzene, heptane, hexane, isobutane, isopropanol, methanol, pentane, propane, toluene, m-xylene, o-xylene, and p-xyleneheptane; and
- the bitter acids concentrate comprises less than 5% of iso-alpha acids.

The advantages of the bitter acids concentrate according to the present invention have been disclosed in relation to the method according to the first aspect of the present invention and are not herewith repeated.

In a preferred embodiment, the bitter acids concentrate according to the invention comprises more than 60% by weight, with respect to the total dry weight of the concentrate, of alpha-acids and not more than 3% by weight, with respect to the total dry weight of the concentrate, of beta-acids.

In a further preferred embodiment, the bitter acids concentrate according to the invention comprises more than 60% by weight, with respect to the total dry weight of the concentrate, of alpha-acids and not more than 0.0001% per cent by weight of one or more organic solvents selected from a group consisting of acetone, benzene, butane, chloroform, cyclohexane, dichloromethane, ethanol, ethyl acetate, ethylbenzene, heptane, hexane, isobutane, isopropanol, methanol, pentane, propane, toluene, m-xylene, o-xylene, and p-xyleneheptane.

Thanks to its compositional properties, said bitter acids concentrates might be advantageously used in a brewing process and/or for preparing a pharmaceutical product, a nutraceutical product, a cosmetic product, a food product, a feed product, an antimicrobial, an antibacterial, an insecticide, or a biopesticide containing bitter acids.

The present invention therefore refers in a further aspect to a use of the bitter acids concentrate according to the present invention in a brewing process, and, in a still further aspect, it refers also to a method for preparing a pharmaceutical product, a nutraceutical product, a cosmetic product, a food product, a feed product, an antimicrobial, an antibacterial, an insecticide, a biopesticide comprising one or more bitter acids, said method comprising the steps of:
- providing a bitter acids concentrate according to the present invention and/or preparing a bitter acids concentrate according to the method according to the first aspect of the invention; and
- obtaining a pharmaceutical product, a nutraceutical product, a cosmetic product, a food product, a feed product, an antimicrobial, an antibacterial, an insecticide, or a biopesticide comprising one or more bitter acids.

The advantages of these use and methods according to the present invention have been disclosed in relation to the method according to the first aspect of the present invention and are not herewith repeated.

The bitter acids concentrate according to the method can be utilized for the production of beer without modifications to the traditional brewing process as it were using CO₂ extract. The formula does not require change in the quantity or type of malt to obtain and maintain a pH between 4 and 5 throughout the brewing process to obtain a beer with a alcohol content between 5 and 6% weight by weight.

### EXPERIMENTAL PART

### Example 1

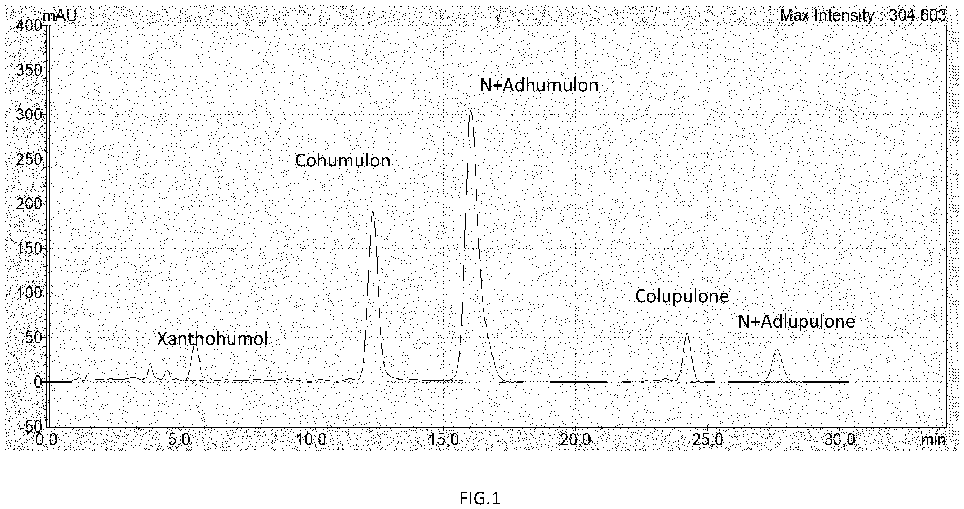

A refined sunflower oil based soluble extract ("Lipid extract") obtained according to the method indicated in patent US10973864B2, wherein the ratio vegetable oil to plant material (Chinook hops having a alpha acids content of 13% and a beta acids content of 3.5% by weight dry matter) is 2.5 to 1, and having the composition reported in Table 1 as determined by HPLC analysis (Figure 1), was provided.

**Table 1**

| **% by weight** | **Alpha acids** | **Beta acids** | **Iso-alpha** | **Xanthohum ol** | **Essential oil** |
|---|---|---|---|---|---|
| Lipid extract | 3.77 | 0.6 | < LOQ | 0.04 | 1.2%* |

| | | | | | |
|---|---|---|---|---|---|
| *Determinations of the total essential oil content were carried out according to Analytica-EBC 7.10 method 26. | | | | | |

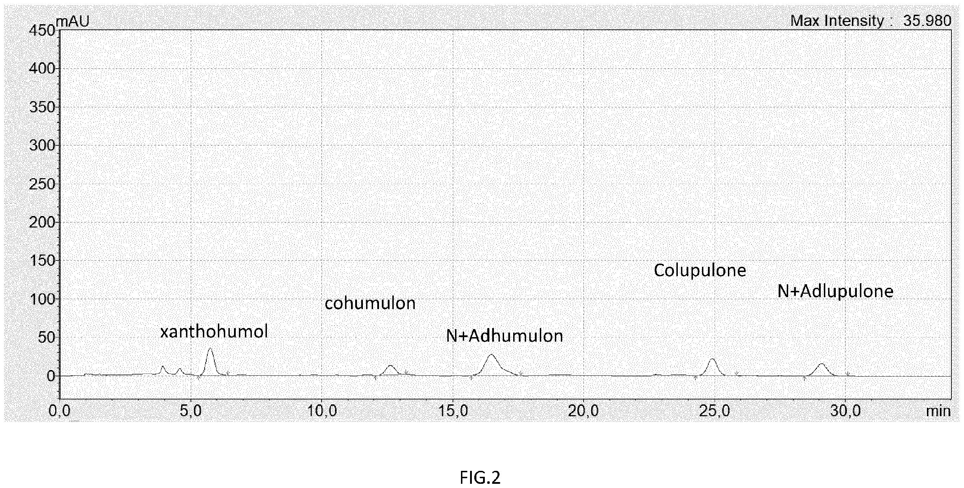

100 g of said extract were mixed with 200 g of an aqueous solution 0.05 M KOH at room temperature in a beaker, so to reach a pH of about 9.5. The mixture was kept in agitation for about 15 min. After mixture centrifugation (4.000 rpm for 5 min), 93 g of an oily lighter phase and 204 ml of a heavier aqueous phase were recovered. Samples of the lighter oily phase was taken for HPLC analysis (Figure 2).

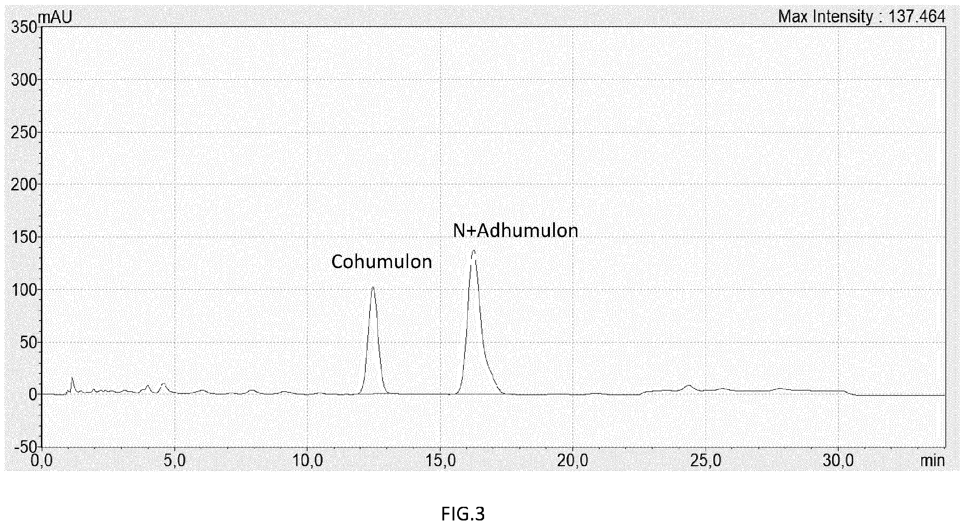

The aqueous phase was acidified utilizing a solution of HCI at 36% concentration to reach a pH of about 1.5-2 so to precipitate the bitter acids. These were recovered as a precipitate after centrifugation (4000 rpm for 5 minutes). The bitter acids precipitate (4.56g) was sampled and the sample sent out for HPLC analysis (Figure 3).

The quantitative determination of iso-alpha, alpha and beta-acids was performed by HPLC-DAD Shimadzu Nexera XR, equipped with a reverse phase C18 column EC 125 x 4 mm Nucleodur 100-5, 5 µm Macherey-Nagel. The analytical method was in accordance with the European Reference Analytical Method EBC 7.11.

Chromatographic conditions: Solvent A: Methanol; Solvent B: Methanol:water:85% phosphoric acid 75:24:1 (% v/v). Flow: 1 ml/min. Oven temperature: 35 °C. Manual injection: loop 20 µL. Detection wavelength: 270 nm for iso-alpha acids; 314 nm for alpha- and beta-acids. Gradient elution: 100% of B up to 21.00 min, 65% of B 21.01 to 29 min, 100% of B 29.01 to 34.00 min.

Retention times: isocohumulone about 6.3 minutes; isohumulone about 8.2 minutes; isoadumulone about 8.8 minutes; cohumulone about 12.6 minutes; N+Adhumulone about 16.5 minutes; Colupulone about 24.9 minutes; N+Adlupulone about 29.0 minutes.

The quantification of said analytes was performed by an external standard method through the preparation of a calibration curve, using ICS-X1 (internal calibration standard-X1) as xanthohumol standard, ICE-4 (international calibration extract-4) as alpha- and beta- acids standard and ICS-I4 (international calibration standards-14) as iso alpha acids standard.

The data are reported as total iso alpha-, alpha- and beta- acids and xanthohumol.

The range of the calibration curve for each group of analytes and for xanthohumol is reported:

**Table 2**

| | | |
|---|---|---|
| **XNT** | 9,6 | 480 |
| **iso-alpha** | 13,3008 | 665,04 |
| **alpha** | 45,21996 | 2260,998 |
| **beta** | 28,18548 | 1409,274 |

The instrumental lower limit of quantification (LOQ) for each analyte is reported:

**Table 3**

| | **LOQ** |
|---|---|
| **XNT** | 5,12 |
| **iso-alpha** | 22,95062 |
| **alpha** | 14,30506 |
| **beta** | 6,52 |

LOQ values of the analytical method, for each analyte and for each matrix, is reported:

**Table 4**

| | | |
|---|---|---|
| Lipid extract | XNT | 0,02% |
| | iso-alpha | 0,08% |
| | alpha | 0,05% |
| | beta | 0,02% |
| Lighter oily phase | XNT | 0,02% |
| | iso-alpha | 0,08% |
| | alpha | 0,05% |
| | beta | 0,02% |
| Precipitate from aqueous phase | | |
| | XNT | 0,01% |
| | iso-alpha | 0,02% |
| | alpha | 0,01% |
| | beta | 0,01% |

The following concentrations (% w/w) of alpha and beta-acids in the various fractions obtained are reported:

**Table 5**

| **% by dry weight** | **Alpha acids** | **Beta acids** | **Iso-alpha** | **Xanthoh umol** | **Essentia I oil*** |
|---|---|---|---|---|---|
| Lighter oily phase | 0.38 | 0.32 | < LOQ | 0.04 | 1.1% |
| Precipitate from aqueous phase | 68.23 | 2.11 | < LOQ | < LOQ | 0.2% |

| | | | | | |
|---|---|---|---|---|---|
| *Determinations of the total essential oil content were carried out according to Analytica-EBC 7.10 method 26. | | | | | |

As it can be seen, achieving a pH of 9.5 allows for efficiently obtaining a concentrate having a high bitter acids content as well as a ratio alpha acids to beta acids greatly in favor of the alpha acids.

### Example 2

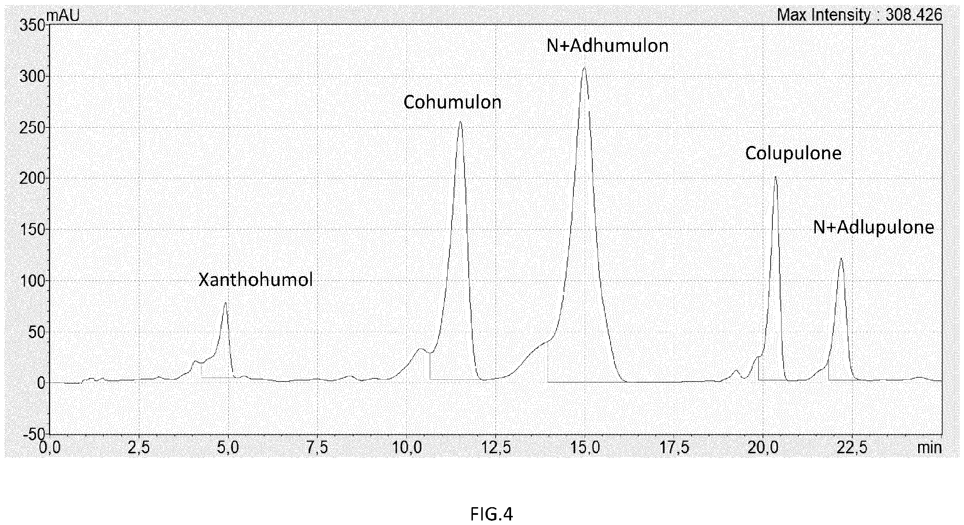

A refined sunflower oil based soluble extract ("Lipid extract") obtained according to the method indicated in patent US10973864B2, wherein the ratio vegetable oil to plant material dry matter (Chinook hops) is 2.5 to 1, and having the composition reported in Table 1 as determined by HPLC analysis (Figure 4), was provided.

**Table 6**

| **% by weight** | **Alpha acids** | **Beta acids** | **Iso-alpha** | **Xanthohumol** |
|---|---|---|---|---|
| Lipid extract | 4.23 | 1.33 | < LOQ | 0.23 |

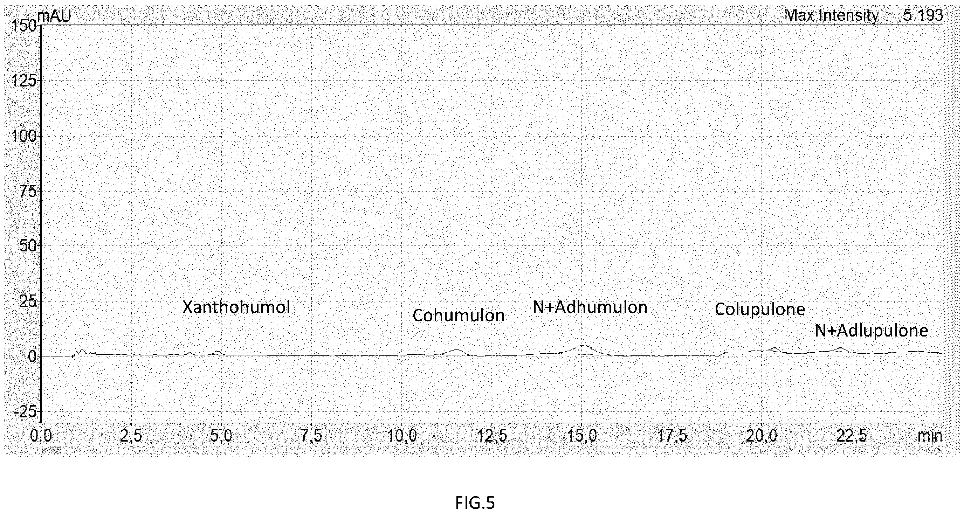

100 g of said extract were mixed with 200 g of an aqueous solution 0.1 M KOH at room temperature in a beaker, so to reach a pH of about 12. The mixture was kept in agitation for about 5 min. After mixture centrifugation (4.000 rpm for 5 min), 88 g of an oily lighter phase and 203 ml of a heavier aqueous phase were recovered. Samples of the lighter oily phase was taken for HPLC analysis (Figure 5).

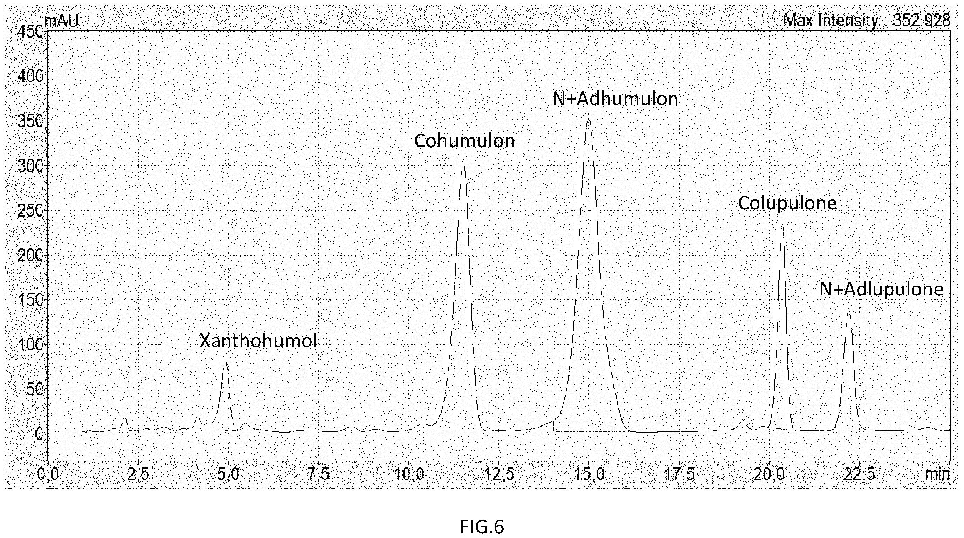

The aqueous phase was acidified utilizing a solution of HCI at 36% concentration to reach a pH of about 1.5-2 so to precipitate the bitter acids. These were recovered as a precipitate after centrifugation (4000 rpm for 5 minutes). The bitter acids precipitate (8.64 g) was sampled and the sample sent out for HPLC analysis (Figure 6).

The quantitative determination of iso-alpha, alpha, beta-acids and xanthoumol was performed by HPLC-DAD Shimadzu Nexera XR, equipped with a reverse phase C18 column EC 125 x 4 mm Nucleodur 100-5, 5 µm Macherey-Nagel. The analythical method was an adjustment of the European Reference Analytical Methods EBC 7.11 and 7.15, validated to reduce the time of the analysis.

Chromatographic conditions: Solvent A: Methanol; Solvent B: Methanol:water:85% phosphoric acid 75:24:1 (% v/v). Flow: 1 ml/min. Oven temperature: 40 °C. Manual injection: loop 20 µL. Detection wavelength: 270 nm for iso-alpha acids; 314 nm for alpha- and beta-acids, 370 nm for xanthohumol. Gradient elution: 100% of B up to 17.5 min, 65% of B 17.51 to 25 min, 100% of B 25.01 min.

Retention times: xanthohumol about 5.0 minutes; isocohumulone about 5.8 minutes; isohumulone about 7.2 minutes; isoadumulone about 7.7 minutes; cohumulone about 12.0 minutes; N+Adhumulone about 15.7 minutes; Colupulone about 20.7 minutes; N+Adlupulone about 22.6 minutes.

The quantification of said analytes was performed as described in Example 1.

**Table 7**

| **% by dry weight** | **Alpha acids** | **Beta acids** | **Iso-alpha** | **Xanthohumol** |
|---|---|---|---|---|
| Lighter oily phase | <LOQ | <LOQ | < LOQ | <LOQ |
| Precipitate from aqueous phase | 51.25 | 15.38 | < LOQ | 1.98 |

As it can be seen, the achievement of a higher pH in the mixture allows to obtain a concentrate having a ratio alpha to beta acids similar to the original plant material.

## Claims

1. A method for preparing a bitter acids concentrate comprising more than 50% by dry weight, with respect to the total dry weight of the concentrate, of bitter acids, comprising the steps of:
a) providing a lipid extract from a starting biological material containing bitter acids;
b) mixing said lipid extract with an alkaline aqueous solution to form a mixture having a pH of at least 9;
c) separating from the mixture of step b) an aqueous phase containing hop bitter acid salts; and
d) obtaining said bitter acids concentrate.

2. The method according to claim 1, wherein the lipid extract of step a) comprises at least 3 % dry weight, with respect to the lipid extract dry weight, of bitter acids and in said lipid extract the weight ratio (alfa acids) : (beta acids) is of more than 1:1, and wherein in the resulting bitter acids concentrate the weight ratio (alpha acids) : (beta acids) is of at least 3:1.

3. The method according to claim 1 or 2, wherein the lipid extract of step a) comprises at least 3 % by dry weight, with respect to the lipid extract dry weight, of bitter acids and at least 0.2% by of xanthohumol, wherein the mixture of step b) has a pH of at least 11, and wherein the resulting bitter acids concentrate comprises at least 1.5% by dry weight, with respect to the total dry weight of said concentrate, of xanthohumol.

4. The method according to any one of claims 1-3, wherein the alkaline aqueous solution of step b) comprises at least one hydroxide of at least one metal selected from the group consisting of: alkali metal, and alkaline earth metal.

5. The method according to any one of claims 1-4, wherein in step b) the alkaline aqueous solution is added to the lipid extract in a weight ratio (alkaline aqueous solution) : (lipid hop extract) of at least 2:1.

6. The method according to any one of claims 1-5, wherein in step b) the alkaline aqueous solution has a molarity of NaOH calculated on the base of the total acidity of the lipid extract expressed as moles of KOH required for acidic titration of the lipid extract, so that the ratio NaOH mol / KOH mol is in the range of 5-12.

7. The method according to any one of claims 1-6, wherein the mixture of step b) is mixed at 25 °C for a time in the range from at least 5 seconds to less than 60 minutes.

8. The method according to any one of claims 1-7, wherein the mixture of step b) has a pH value of at most 13.5.

9. The method according to any one of claims 1-8, wherein before step c) at least one salt and/or at least one sugar is added to the mixture of step b).

10. The method according to any one of claims 1-9, wherein said step c) of separating comprises separating from the mixture of step b):
- a lighter oily phase, and
- a heavier aqueous phase, wherein the heavier aqueous phase is the aqueous phase containing bitter acid salts.

11. The method according to claim 10, wherein the lighter oily phase is distilled to recover hops low-boiling terpenes.

12. The method according to any one of claims 1-11, wherein said step d) comprises:
i. acidifying the aqueous phase of said step c) to a pH of less than 4, thus forming a precipitate comprising bitter acids;
ii. separating the precipitate of step i. from the remaining aqueous phase, wherein said precipitate is said bitter acids concentrate.

13. The method according to anyone of claims 1-11, wherein said step d) comprises:
i. modifying the pH of the aqueous phase of said step c) to a value ranging from 12.5 to 9, thus forming a first precipitate;
ii. separating the first precipitate of step i. from the remaining aqueous phase, wherein said first precipitate contains impurities;
iii. modifying the pH of the remaining aqueous phase of step ii. to a value of less than 11.5, thus forming a second precipitate;
iv. separating the second precipitate from the further remaining aqueous phase, wherein said second precipitate is said bitter acids concentrate.

14. The method according to any one of claims 1-11, wherein said step d) comprises drying the aqueous phase, thus forming a dried product, wherein said dried product is said bitter acids concentrate.

15. The method according to any one of claims 1-14, wherein said bitter acids concentrate comprises less than 10 %, by weight on total concentrate dry weight, of total beta acids.

16. The method according to any one of claims 1-15, wherein said bitter acids concentrate has a beta acids content lower than that of the lipid extract of step a).

17. The method according to any one of claims 1-16, wherein said bitter acids concentrate has a beta acids content lower than that of the starting biological material.

18. A bitter acids concentrate obtainable by means of the method according to any one of claims 1-17.

19. A bitter acids concentrate comprising more than 50% by dry weight, with respect to the total dry weight of the concentrate, of bitter acids and one or more of the following features:
- a weight ratio (alpha acids) : (beta acids) of at least 3:1, preferably of more than 10:1, even more preferably of more than 20:1;
- the bitter acids concentrate comprises at least, preferably more than, 1.5% by dry weight, with respect to the total dry weight of said concentrate, of xanthohumol;
- the bitter acids concentrate comprises less than 10 % by weight, preferably less than 5% by weight, more preferably less than 3% by weight, on total concentrate dry weight, of total beta acids;
- the bitter acids concentrate comprises at least 30% by weight, more preferably at least 50% by weight, even more preferably at least 60% by weight, even more preferably at least 65 % by weight, with respect to the total dry weight of the concentrate, of alpha-acids;
- a content of essential oil, such as humulene, myrcene, and caryophyllene, of less than 1% by weight;
- the bitter acids concentrate has a beta acids content lower than that of the starting biological material;
- the bitter acids concentrate comprises not more than 0.0001% per cent by weight of one or more organic solvents selected from a group consisting of acetone, benzene, butane, chloroform, cyclohexane, dichloromethane, ethanol, ethyl acetate, ethylbenzene, heptane, hexane, isobutane, isopropanol, methanol, pentane, propane, toluene, m-xylene, o-xylene, and p-xyleneheptane; and
- the bitter acids concentrate comprises less than 5% of iso-alpha acids.

20. A method for preparing a pharmaceutical product, a nutraceutical product, a cosmetic product, a food product, a feed product, an antimicrobial, an antibacterial, an insecticide, a biopesticide comprising one or more bitter acids, said method comprising the steps of:
- providing a bitter acids concentrate according to claim 18 or 19 and/or preparing a bitter acids concentrate according to any one of claims 1-17; and
- obtaining a pharmaceutical product, a nutraceutical product, a cosmetic product, a food product, a feed product, an antimicrobial, an antibacterial, an insecticide, or a biopesticide comprising one or more bitter acids.

21. A use of the bitter acids concentrate according to claim 18 or 19 and/or prepared according to any one of claims 1-17, in a brewing process.
